# EUROPEAN PATENT SPECIFICATION

(11) **EP 1 622 915 B1**
(45) Date of publication and mention of the grant of the patent: **06.08.2008**
(21) Application number: 04759279.5
(22) Date of filing: 08.04.2004
(51) Int. Cl.: C07D 495/04, C11B 9/00

(54) **FRAGRANCE COMPOSITIONS COMPRISING BENZO ¬4,5 THIENO¬3,2-B PYRAN-2-ONE**
BENZO ¬4,5 THIENO¬3,2-B PYRAN-2-ON ENTHALTENDE DUFTSTOFFZUSAMMENSETZUNGEN
COMPOSITIONS DE FRAGRANCE COMPRENANT DU BENZO¬4,5 THIENO¬3,2-B PYRAN-2-ONE

(30) Priority: 08.04.2003 US 461090 P
(43) Date of publication of application: 08.02.2006
(73) Proprietor: Flexitral, Inc., Chantilly, VA 20151 (US)
(72) Inventor: TURIN, Luca, London NW1 7NB (GB)
(74) Representative: Crowhurst, Charlotte Waveney
(86) International application number: PCT/US2004/010829
(87) International publication number: WO 2004/092182

(56) References cited:
- MUSTAFA A ET AL: "Synthesis of Substituted Linear Furano[2,3-g][1]benzopyrones and [3,2-b]Thianaphthenones" TETRAHEDRON, ELSEVIER SCIENCE PUBLISHERS, AMSTERDAM, NL, vol. 21, 1965, pages 849-859, XP002110139 ISSN: 0040-4020
- BUGGLE ET AL.: "Some Reactions of 2H-[1]Benzothieno[3,2-b]pyran-2-ones and Related Compounds" MONATSHEFTE FÜR CHEMIE, vol. 119, 1988, pages 945-951, XP009035612 ISSN: 0040-4020

## Description

The present invention relates generally to the field of flavourings and fragrances. More particularly, the present invention relates to the use of beazo[4,5]thieno[3,2b]pyran-2-one as a flavour or fragrance, and to perfumes and other fragrant articles comprising this compound.

Many aromachemicals are used in the flavouring and fragrance industries. For example, citral has a lemon scent and as such is used as a flavour and/or fragrance in many articles of manufacture. However, many aromachemicals include isoprene units and/or benzene rings which are potentially susceptible to reaction and may result in a limited useful lifetime. Further, many essential oil fragrances have recently been determined to cause allergic reactions, and it is becoming increasingly difficult to bring such compounds to market.

Many aromachemicals, which are fundamental to the formation of various fragrances, have been placed on the allergens list and are being banned or restricted in many commercial regions. The bans or restrictions will undoubtedly have a considerable effect on the quality of various fragrances, largely because the reduction in the perfumers' palette makes the creation of certain notes virtually impossible.

It would be desirable to develop derivatives of aromachemicals that include benzene rings and/or isoprene units that do not similarly result in allergic reactions and/or which have improved useful lifetimes or other beneficial properties. Such additionally beneficial properties include improved odour intensity and stability. The present invention provide such fragrances and flavourings.

Buggle et al., Monatshefte für Chemie 119 (1988), 945-951 describes the use of benzo[4,5]thieno[3,2-b]pyran-2-one as an intermediate in a thiation reaction. There is no disclosure of the flavour or fragrance properties of this compound.

The present invention provides the use of benzo[4,5]thieno[3,2b]pyran-2-one as a flavour or fragrance.

A method to improve, enhance or modify the odour of a perfuming composition or a perfumed article comprising adding to the composition or the article an effective amount of benzo[4,5]thieno[3,2b]pyran-2-one is also provided.

A method to improve, enhance or modify the flavour of a beverage, a food, a pharmaceutical composition, an orally-deliverable matrix material or other orally-delivered product comprising adding thereto an effective amount of benzo[4,5]thieno[3,2b]pyran-2-one is provided.

The present invention provides a composition or article comprising benzo[4,5]thieno[3,2b]pyran-2-one and at least one other perfuming ingredient, solvent, or adjuvant suitable for use in perfumery.

A perfume or cologne, a soap, a bath or shower gel, a shampoo or other hair care product, a cosmetic preparation, a body deodorant or antiperspirant, an air freshener, a fabric detergent or softener or an all-purpose household cleaner comprising benzo[4,5]thieno[3,2b]pyran-2-one is also provided.

The following products are also provided a body deodorant or an antiperspirant comprising benzo[4,5]thieno[3,2b]pyran-2-one as a perfuming ingredient, a detergent comprising benzo[4,5]thieno[3,2b]pyran-2-one, a bleach composition comprising benzo[4,5]thieno[3,2b]pyran-2-one, a beverage composition comprising benzo[4,5]thieno[3,2b]pyran-2-one, and a food, pharmaceutical composition, orally-deliverable matrix material or other orally-delivered product comprising benzo[4,5]thieno[3,2b]pyran-2-one.

Examples of suitable articles of manufacture include candles, air fresheners, perfumes, disinfectant compositions, hypochlorite (bleach) compositions, beverages such as beer and soda, denture cleanser tablets as described, for example, in US Patent No. 5,571,519, and flavoured orally-delivered products such as lozenges, candies, and the like.

Benzo[4,5]thieno[3,2b]pyran-2-one can be included in virtually any article of manufacture that can include the aromachemicals or other "parent compounds" from which it is derived. Examples include bleach, detergents, flavourings and fragrances, beverages, including alcoholic beverages, and the like. Benzo[4,5]thieno[3,2b]pyran-2-one can be used in applications like soaps, shampoos, body deodorants and antiperspirants, solid or liquid detergents for treating textiles, fabric softeners, detergent compositions and/or all-purpose cleaners for cleaning dishes or various surfaces, for both household and industrial use. Of course, the use of benzo[4,5]thieno[3,2b]pyran-2-one is not limited to the above-mentioned products, as it can be used in other current uses in perfumery, namely the perfuming of soaps and shower gels, hygiene or hair-care products, as well as of body deodorants, air freshners and cosmetic preparations, and even in fine perfumery, namely in perfumes and colognes. These uses are described in more detail below.

### Perfume Compositions

Benzo[4,5]thieno[3,2b]pyran-2-one can be used as a perfuming ingredient, preferably at a range of at least about 30% by weight of the perfume composition, more preferably at a range of at least about 60% by weight of the composition. Benzo[4,5]thieno[3,2b]pyran-2-one can even be used in its pure state or as a mixture, without added components. The olfactive characteristics of benzo[4,5]thieno[3,2b]pyran-2-one are also present in mixtures thereof, and mixtures of these compounds can be used as perfuming ingredients. This may be particularly advantageous where separation and/or purification steps can be avoided by using compound mixtures.

In all cited applications, benzo[4,5]thieno[3,2b]pyran-2-one can be used alone or in admixture with other perfuming ingredients, solvents or adjuvants of current use in the art. The nature and the variety of these co-ingredients do not require a more detailed description here, which, moreover, would not be exhaustive, and the person skilled in the art will be able to choose the latter through general knowledge and as a function of the nature of the product to be perfumed and of the desired olfactive effect.

These perfuming ingredients typically belong to chemical classes as varied as alcohols, aldehydes, ketones, esters, ethers, acetates, nitrites, terpene hydrocarbons, sulfur- and nitrogen-containing heterocyclic compounds, as well as aromachemicals of natural or synthetic origin. Many of these ingredients are described in reference textbooks such as the book of S. Arctander, Perfume and Flavour Chemicals, 1969, Montclair, N.J., USA, or in its more recent versions, or in other works of similar nature.

The proportions in which benzo[4,5]thieno[3,2b]pyran-2-one can be incorporated in the various products vary within a large range of values. These values depend on the nature of the article or product that one desires to perfume and the odour effect searched for, as well as on the nature of the co-ingredients in a given composition when the compounds are used in admixture with perfuming co-ingredients, solvents or adjuvants of current use in the art.

As an example, benzo[4,5]thieno[3,2b]pyran-2-one is typically present at concentrations between about 0.1 and about 10%, or even more, by weight of relative to the weight of the perfuming composition in which it is incorporated. Far lower concentrations than those mentioned above can be used when benzo[4,5]thieno[3,2b]pyran-2-one is directly applied for perfuming the various consumer products cited beforehand.

Benzo[4,5]thieno[3,2b]pyran-2-one is relatively stable in typically aggressive media for perfumes. Accordingly, it can be used in detergents containing bleaching agents and activators such as, for example, tetraacetylethlenediamine (TAED), hypohalites, in particular hypochlorite, peroxygenated bleaching agents such as, for example, perborates, etc. Benzo[4,5]thieno[3,2b]pyran-2-one can also be used in body deodorants and antiperspirants, for example, those containing aluminum salts. These embodiments are described in more detail below.

### Conventional Detergent Ingredients

In addition to benzo[4,5]thieno[3,2b]pyran-2-one compositions may include a detersive surfactant and optionally, one or more additional detergent ingredients, including materials for assisting or enhancing cleaning performance, treatment of the substrate to be cleaned, or to modify the aesthetics of the detergent composition (e.g., perfumes, colorants, dyes, etc.). The following are illustrative examples of detersive surfactants and other detergent ingredients.

Detersive Surfactants. Non-limiting examples of synthetic detersives surfactants useful herein, typically at levels from about 0.5% to about 90%, by weight, include the conventional C₁₁₋₁₈ alkyl benzene sulfonates ("LAS") and primary, branch-chain and random C₁₀₋₂₀ alkyl sulfates ("AS"), the C₁₀₋₁₈ secondary (2,3) alkyl sulfates of the formula CH₃(CH₂)ₓ(CH(CH₃)OSO₃⁻M⁺) and CH₃(CH₂)_{y}(CH(CH₂CH₂)OSO₃⁻M⁺) wherein x and y are integers and wherein each of x and (y+1) is least about 7, preferably at least about 9, and M is a water-solubilizing cation, especially sodium, unsaturated sulfates such as oleyl sulphate, the C₁₀₋₁₈ alkyl alkoxy sulfates ("AEx S"; especially EO 1-7 ethoxy sulfates), C₁₀₋₁₈ alkyl alkoxy carboxylates (especially the EO 1-5 ethoxycarboxylates), the C₁₀₋₁₈ glycerol ethers, the C₁₀₋₁₈ alkyl polyglycosides and their corresponding sulphated polyglycosides, and C₁₂₋₁₈ alpha-sulfonated fatty acid esters. If desired, the conventional non-ionic and amphoteric surfactants such as the C₁₂₋₁₈ alkyl ethoxylates ("AE") including the so-called narrow peaked alkyl ethoxylates and C₆₋₁₂ alkyl phenol alkoxylates (especially ethoxylates and mixed ethoxy/propoxylates), C₁₂₋₁₈ betaines and sulfobetaines ("sultaines"), C₁₀₋₁₈ amine oxides, and the like, can also be included in the overall compositions. The C₁₀₋₁₈ N-alkyl polyhydroxy fatty acid amides can also be used. Typical examples include the C₁₂₋₁₈ N-methylglucamides. See WO 92/06154. Other sugar-derived surfactants include the N-alkoxy polyhydroxy fatty acid amides, such as C₁₀₋₁₈ N-(3-methoxypropyl) glucamide. The N-propyl through N-hexyl C₁₂₋₁₈ glucamides can be used for low sudding. C₁₀₋₂₀ conventional soaps may also be used, however synthetic detergents are preferred. If high sudding is desired, the branched-chain C₁₀₋₁₆ soaps may be used. Mixtures of anionic and non-ionic surfactants are especially useful. Other conventional useful surfactants are listed in standard texts. See also US Patent No. 3,664,961 to Norris.

Preferred compositions incorporating only synthetic detergents have a detergent level of from about 0.5% to 50%. Compositions containing soap preferably comprise from about 10% to about 90% soap.

Although the detergent compositions herein can consist of only detersive surfactant and Benzo[4,5]thieno[3,2b]pyran-2-one, the said compositions preferably contain other ingredients commonly used in detergent products.

### Builders

Detergent builders can optionally be included in the compositions herein to assist in controlling mineral hardness. Inorganic as well as organic builders can be used. Builders are typically used in fabric laundering compositions to assist in the removal of particulate soils.

The level of builder can vary widely depending upon the end of the composition and its desired physical form. When present, the compositions will typically comprise at least about 1% builder. Liquid formulations typically comprise from about 5% to about 50%, more typically about 5% to about 30%, by weight, of detergent builder. Granular formulations typically comprise from about 10% to about 80%, more typically from about 15% to about 50% by weight, of the detergent builder. Lower or higher levels of builder, however, are not meant to be excluded.

Inorganic or detergent builders include, but are not limited to phosphate builder such as, the alkali metal, ammonium and allanolammonium salts of polyphosphates (exemplified by the tripolyphosphates, pyrophosphates, and glassy polymeric meta-phosphates), phosphonates, and phytic acid, and non-phosphorous builders such as silicates, carbonates (including bicarbonates and sesquicarbonates), sulphates and aluminosilicates. Non-phosphate builders are required in some locales.

Organic builders suitable for use herein include polycarboxylate builders such as disclosed in US Patent No. 3,308,067 to Diehl; US Patent No. 4,144,226 to Crutchfield and US Patent No. 4,246,495 to Crutchfield.

### Soil Release Agents

Soil release agents are desirably used in laundry detergents of the instant invention. Suitable soil release agents include those of US Patent No. 4,968,451 to Scheibel and Gosselink. Such ester oligomers can be prepared by (a) ethoxylating allyl alcohol, (b) reacting the product of (a) with dimethyl terephthalate ("DMT") and 1,2-propylene glycol ("PG") in a two-stage transesterification/oligomerization procedure and (c) reacting the product of (b) with sodium metabisulfite in water; the non-ionic end-capped 1,2-propylene/polyoxyethylene terephthalate polyesters of US Patent No. 4,711,730 to Gosselink et al., for example those produced by transesterfication/oligomerization of poly(ethyleneglycol) methyl ether, DMT, PG and poly(ethyleneglycol) ("PEG"); the partly- and fully-anionic-end-capped oligomeric esters of US Patent No. 4,721,580 to Gosselink, such as oligomers from ethylene glycol ("EG"), PG, DMT and Na-3,6-dioxa-8-hydroxyoctanesulfonate; the non-ionic-capped block polyester oligomeric compounds of US Patent No. 4,702,857 to Gosselink, for example produced from DMT, Me-capped PEG and EG and/or PG, or a combination of DMT, EG and/or PG, Me-capped PEG and Na-dimethyl-5-sulfoisophthalate; and the anionic, especially sulfoaroyl, end-capped terephthalate esters of US Patent No: 4,877,896 to Maldonado, Gosselink et al., the latter being typical of SRA's useful in both laundry and fabric conditioning products, an example being an ester composition made from m-sulfobenzoic acid monosodium salt, PG and DMT optionally but preferably further comprising added PEG, e.g., PEG 3400. Another preferred soil release agent is a sulfonated end-capped type described in US Patent No. 5,415,807.

### Other Optional Ingredients

The compositions herein can contain other ingredients such as enzymes, bleaches, fabric softening agents, dye transfer inhibitors, suds suppressors, and chelating agents, all well known within the art.

For purposes of defining detergent compositions of the present invention, the pH of the detergent composition is that which is measured at 1% concentration of the detergent composition in distilled-water at 20°C. The detergent compositions herein have a pH of from about 7.1 to about 3, more typically from about 7.5 to about 9.5 for liquid detergents and from about 8 to about 12 for granular detergents.

### Formulation with Detergents With or Without Conventional Perfumery Materials

While benzo[4,5]thieno[3,2b]pyran-2-one can be used alone and simply mixed with essential detergent ingredients, most notably surfactant, it can also be desirably combined into three-part formulations which contain a non-fragranced detergent base comprising one or more synthetic detergents. In one embodiment, both aldehydes and acetals are present, such that the aldehydes provide desirable in-package and in-use (wash-time) fragrance, while the acetals provide a long-term fragrance to the laundered textile fabrics.

In formulating the present detergents, the fully-formulated fragrance can be prepared using numerous known odorant ingredients of natural or synthetic origin. The range of the natural raw substances can embrace not only readily-volatile, but also moderately-volatile and slightly-volatile components and that of the synthetics can include representatives from practically all classes of fragrant substances, as will be evident from the following illustrative compilation: natural products, such as tree moss absolute, basil oil, citrus fruit oils (such as bergamot oil, mandarin oil, etc.), mastix absolute, myrtle oil, palmarosa oil, patchouli oil, petitgrain oil Paraguay, wormwood oil, alcohols, such as farnesol, geraniol, linalool, nerol, phenylethyl alcohol, rhodinol, cinnamic alcohol, aldehydes, such as citral, Helional^{™}, alpha-hexyl-cinnamaldehyde, hydroxycitronellal, Lilial^{™} (p-tert-butyl-alpha-methyldihydrocinnamaldehyde), methylaonylacetaldehyde, ketones, such as allylionone, alpha-ionone, beta-ionone, isoraldein (isomethyl-alpha-ionone), methylionone, esters, such as allyl phenoxyacetate, benzyl salicylate, cinnamyl propionate, citronellyl acetate, citronellyl ethoxolate, decyl acetate, dimethylbenzylcarbinyl acetate, dimethylbenzylcarbinyl butyrate, ethyl acetoacetate, ethyl acetylacetate, hexenyl isobutyrate, linalyl acetate, methyl dihydrojasmonate, styrallyl acetate, vetiveryl acetate, etc., lactones, such as gamma-undecalactone, various components often used in perfumery, such as musk ketone, indole, p-menthane-8-thiol-3-one, and methyl-eugenol. Likewise, any conventional fragrant acetal or ketal known in the art can be added to the present composition as an optional component of the conventionally formulated perfume (c). Such convential fragrant acetals and ketals include the well-known methyl and ethyl acetals and ketals, as well as acetals or ketals based on benzaldehyde, those comprising phenylethyl moieties, or more recently developed specialities such as those described in a United States Patent entitled "Acetals and Ketals of Oxo-Tetralins and Oxo-Indanes, see US Patent No. 5,084,440. Of course, other recent synthetic specialities can be included in the perfume compositions for fully-formulated detergents. These include the enol ethers of alkyl-substituted oxo-tetralins and oxo-indanes as described in US Patent No. 5,332,725; or Schiff Bases as described in US Patent No. 5,264,615. It is preferred that the pro-fragrant material be added separately from the conventional fragrances to the detergent compositions of the invention.

### Formulation with other Special-Purpose Fragrance Delivering Compounds

Detergents including benzo[4,5]thieno[3,2b]pyran-2-one may further, optionally, if desired, contain other known compounds having the capability to enhance substantivity of a fragrance. Such compounds include, but are not limited to, the aluminium alkoxides such as isobutylaluminium diferanylate as disclosed in US Patent No. 4,055,634; or the known titanate and zirconate esters or oligoesters of fragrant materials such as those disclosed in US Patent Nos. 3,947,574 and 3,779,932. When using such organoaluminum, organotitanium or organozinc derivatives, they may be incorporated into the present formulations at their art-known levels.

### Beverage compositions

Benzo[4,5]thieno[3,2b]pyran-2-one can be incorporated into beverages and impart various flavourings to the beverages. The beverage composition can be a cola beverage composition, and can also be coffee, tea, dairy beverage, fruit juice drink, orange drink, lemon-lime drink, beer, malt beverages, or other flavoured beverage. The beverages can be in liquid or powdered form.

The beverage compositions can also include one or more flavouring agents; artificial colourants; vitamin additives; preservatives; caffeine additives; water; acidulants; thickeners; buffering agents; emulsifiers; and or fruit juice concentrates.

Artificial colorants which may be used include caramel colour, yellow 6 and yellow 5.

Useful vitamin additives include vitamin B2, vitamin B6, vitamin B12, vitamin C (ascorbic acid), niacin, pantothenic acid, biotin and folic acid. Suitable preservatives include sodium or potassium benzoate. Salts which may be used include sodium, potassium and magnesium chloride. Exemplary emulsifiers are gum arabic and purity gum, and a useful thickener is pectin. Suitable acidulants include citric, phosphoric and malic acid, and potential buffering agents include sodium and potassium citrate.

In one embodiment, the beverage is a carbonated cola beverage. The pH is generally about 2.8 and the following ingredients can be used to make the syrup for these composition: Flavour Concentrate, including Benzo[4,5]thieno[3,2b]pyran-2-one (22.22 ml), 80% Phosphoric Acid (5.55g), Citric Acid (0.267g), Caffeine (1.24g), artificial sweetener, sugar or corn syrup (to taste, depending on the actual sweetener) and Potassium Citrate (4.07g). The beverage composition can be prepared, for example, by mixing the foregoing syrup with carbonated water in a proportion of 50ml syrup to 250ml of carbonated water.

In another embodiment, the beverage is a beer or malt beverage. Preferred flavourings for beer and malt beverages include lemon, lime and lemon-lime. Advantageously, the flavourings include citral derivatives in which one of both of the double bonds are replaced with a cyclopropane group, where the cyclopropane groups can, independently, be unsubstituted, or include one or two alkyl or substituted alkyl groups, preferably methyl groups. The amount of flavouring can be adjusted according to taste.

### Orally Delivered Products

Flavoured food and pharmaceutical compositions including benzo[4,5]thieno[3,2b]pyran-2-one can also be prepared. Benzo[4,5]thieno[3,2b]pyran-2-one can be incorporated into conventional foodstuffs using techniques well known to those of skill in the art. Alternatively, benzo[4,5]thieno[3,2b]pyran-2-one can be incorporated within polymeric particles, which can, in turn, be dispersed within and/or over a surface of an orally-deliverable matrix material, which is usually a solid or semi-solid substrate. When used in chewable compositions, benzo[4,5]thieno[3,2b]pyran-2-one can be released into the orally-deliverable polymeric matrix material as the composition is chewed and held in the mouth, thus prolonging the flavour of the composition. In the case of dried powders and mixers, the flavour can be made available as the product is consumed or be released into the matrix material as the composition is further processed. When two flavours are combined with the polymeric particles, the relative amounts of the additives can be selected to provide simultaneous release and exhaustion of the compounds.

In one embodiment, the flavoured composition includes an orally-deliverable matrix material; a plurality of water insoluble polymeric particles dispersed in the orally-deliverable matrix material, where the polymeric particles individually define networks of internal pores and are non-degradable in the digestive tract; and one or more derivatives as described herein entrapped within the internal pore networks. The derivatives are released as the matrix is chewed, dissolved in the mouth, or undergoes further processing selected from the group consisting of liquid addition, dry blending, stirring, fixing, heating, baking, and cooking. The orally-deliverable matrix material can be selected from the group consisting of gums, latex materials, crystallized sugars, amorphous sugars, fondants, nougats, jams, jellies, pastes, powders, dry blends, dehydrated food mixes, baked goods, batters, doughs, tablets, and lozenges.

### Time Release Formulations

In one embodiment, benzo[4,5]thieno[3,2b]pyran-2-one is incorporated into a system which can release a fragrance in a controlled manner. These include substrates such as air fresheners, laundry detergents, fabric softeners, deodorants, lotions, and other household items. US Patent No. 4,587,129, describes a method for preparing gel articles which contain up to 90% by weight of fragrance or perfume oils. The gels are prepared from a polymer having a hydroxy (lower alkoxy) 2-alkeneoate, a hydroxy (lower alkoxy) lower alkyl 2-alkeneoate, or a hydroxy poly (lower alkoxy) lower alkyl 2-alkeneote and a polyethylenically unsaturated crosslinking agent. These materials have continuous slow release properties, i.e., they release the fragrance component continuously over a long period of time.

The present invention will be better understood with reference to the following non-limiting example.

### Example 1: Synthesis of benzo[4,5]thieno[3,2b]pyran-2-one

As shown below in Scheme 1, 2-mercaptobenzoic acid was reacted with trans-glutoconic acid in the presence of a catalytic amount of sulphuric acid to form benzo[4,5]thieno[3,2b]pyran-2-one. The chemistry is applicable to the synthesis of other compounds, where the mercapto-benzoic acid is further substituted with functional groups, such as alkyl groups, that do not interfere with the cyclization chemistry. Functional groups that otherwise might interfere with the cyclization chemistry can be present, provided they are protected. Protecting groups for such functional groups (i.e., hydroxy, amine, thiol and the like) are well known in the art and need not be discussed herein.

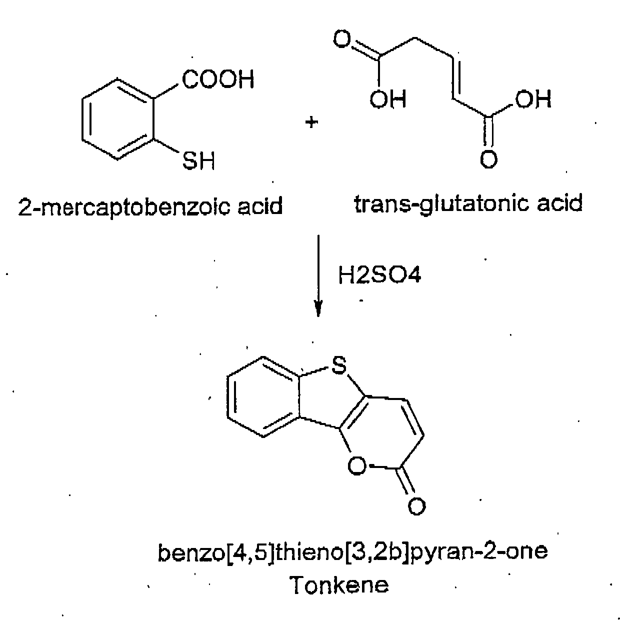

## Claims

1. The use of benzo[4,5]thieno[3,2b]pyran-2-one as a flavour or fragrance.

2. A method to improve, enhance or modify the odor of a perfuming composition or a perfumed article comprising adding to the composition or the article an effective amount of benzo[4,5]thieno[3,2b]pyran-2-one.

3. A method according to claim 2, wherein the benzo[4,5]thieno[3,2b]pyran-2-one is present in admixture with at least one other perfuming ingredient, solvent, or adjuvant suitable for use in a flavouring and/or fragrance.

4. A method to improve, enhance or modify the flavour of a beverage, a food, a pharmaceutical composition, an orally-deliverable matrix material or other orally-delivered product comprising adding thereto an effective amount of benzo[4,5]thieno[3,2b]pyran-2-one.

5. A composition or article comprising benzo[4,5]thieno[3,2b]pyran-2-one and at least one other perfuming ingredient, solvent, or adjuvant suitable for use in perfumery.

6. A composition according to claim 5, wherein the benzo[4,5]thieno[3,2b]pyran-2-one is present in an amount that is at least 30 percent by weight of the composition.

7. A composition according to claim 6, wherein the benzo[4,5]thieno[3,2b]pyran-2-one is present in an amount that is at least 60 percent by weight of the composition.

8. A perfume or cologne, a soap, a bath or shower gel, a shampoo or other hair care product, a cosmetic preparation, a body deodorant or antiperspirant, an air freshner, a fabric detergent or softener or an all-purpose household cleaner comprising benzo[4,5]thieno[3,2b]pyran-2-one.

9. A body deodorant or antiperspirant, comprising benzo[4,5]thieno[3,2b]pyran-2-one as a perfuming ingredient.

10. The body deodorant or antiperspirant according to claim 9, wherein the benzo[4,5]thieno[3,2b]pyran-2-one is present in admixture with at least one other perfuming ingredient, solvent, or adjuvant suitable for use in perfumery.

11. A detergent comprising benzo[4,5]thieno[3,2b]pyran-2-one.

12. A detergent according to claim 11, wherein benzo[4,5]thieno[3,2b]pyran-2-one is present in admixture with at least one other perfuming ingredient, solvent, or adjuvant of current use in the art.

13. A bleach composition comprising benzo[4,5]thieno[3,2b]pyran-2-one.

14. A beverage comprising benzo[4,5]thieno[3,2b]pyran-2-one.

15. A beverage according to claim 14, selected from the group consisting of beer, malt liquor, lemonade and cola.

16. A food, pharmaceutical composition, orally-deliverable matrix material or other orally-delivered product comprising benzo[4,5]thieno[3,2b]pyran-2-one.

## Patentansprüche

1. Verwendung von Benzo[4,5]thieno[3,2b]pyran-2-on als Aroma oder Duft.

2. Verfahren zur Verbesserung, Verstärkung oder Modifizierung des Geruchs einer parfümierenden Zusammensetzung oder eines parfümierten Gegenstands, wobei das Verfahren das Versetzen der Zusammensetzung oder des Gegenstands mit einer wirksamen Menge von Benzo[4,5]thieno[3,2b]pyran-2-on umfasst.

3. Verfahren nach Anspruch 2, wobei Benzo[4,5]thieno[3,2b]pyran-2-on in einem Gemisch mit mindestens einen weiteren parfümierenden Bestandteil, Lösemittel oder Adjuvans, die zur Verwendung in einem Aroma und/oder Duft geeignet sind, vorhanden ist.

4. Verfahren zur Verbesserung, Verstärkung oder Modifizierung des Aromas eines Getränks, eines Nahrungsmittels, einer pharmazeutischen Zusammensetzung, eines oral zuführbaren Matrixmaterials oder eines anderen oral zugeführten Produkts, wobei das Verfahren das Versetzen derselben mit einer wirksamen Menge von Benzo[4,5]thieno[3,2b]pyran∼2-on umfasst.

5. Zusammensetzung oder Gegenstand, der Benzo[4,5]thieno[3,2b]pyran-2-on und mindestens einen weiteren parfümierenden Bestandteil, ein Lösemittel oder ein Adjuvans, die zur Verwendung in Parfümeriewaren geeignet sind, umfasst.

6. Zusammensetzung nach Anspruch 5, wobei Benzn[4,5]thieno[3,2b]pyran-2-on in einer Menge vorhanden ist, die mindestens 30 Ges.-% der Zusammensetzung beträgt.

7. Zusammensetzung nach Anspruch 6, wobei Benzo[4,5]thieno[3,2b]pyran-2-on in einer Menge vorhanden ist, die mindestens 60 Gew.-% der Zusammensetzung beträgt.

8. Parfum oder Kölnisch Wasser, Seife, Bade- oder Duschgel, Shampoo oder ein anderes Haarpflegeprodukt, Kosmetikpräparat, Körperdeodorant oder Antitranspirationsmittel, Luftverbesserer, Gewebewaschmittel oder Weichspüler oder Allzweckhaushaltsreiniger, die Benzo[4,5]thieno[3,2b]pyran-2-on umfassen.

9. Körperdeodorant oder Antitranspirationsmittel, das Benzo[4,5]thieno[3,2b]pyran-2-on als parfümierenden Bestandteil umfasst.

10. Körperdeodorant oder Antitranspirationsmittel nach Anspruch 9, wobei Benzo[4,5]thieno[3,2b]pyran-2-on in einem Gemisch mit mindestens einem weiteren parfümierenden Bestandteil, Lösemittel oder Adjuvans, die zur Verwendung in Parfümeriewaren geeignet sind, vorhanden ist.

11. Waschmittel, das Benzo[4,5]thieno[3,2b]pyran-2-on umfasst.

12. Waschmittel nach Anspruch 11, wobei Benzo[4,5]thieno[3,2b]pyran-2-on in einem Gemisch mit mindestens einem weiteren parfümierenden Bestandteil, Lösemittel oder Adjuvans, die derzeit auf dem Gebiet verwendet werden, vorhanden ist.

13. Bleichzusammensetzung, die Benzo[4,5]thieno[3,2b]pyran-2-on umfasst.

14. Getränk, das Benzo[4,5]thieno[3,2b]pyran-2-on umfasst.

15. Getränk nach Anspruch 14, das aus der Gruppe von Bier, Starkbier, Limonade und Cola ausgewählt ist.

16. Nahrungsmittel, pharmazeutische Zusammensetzung, oral zuführbares Matrixmaterial oder ein anderes oral zugeführtes Produkt, das Benzo[4,5]thieno[3,2b]pyran-2-on umfasst.

## Revendications

1. Utilisation de la benzo[4,5]thiéno[3,2b]pyran-2-one en tant qu'arôme ou fragrance.

2. Procédé pour améliorer, renforcer ou modifier l'odeur d'une composition de parfum ou d'un article parfumé comprenant l'addition à la composition ou à l'article d'une quantité efficace de benzo[4,5]thiéno[3,2b]pyran-2-one.

3. Procédé selon la revendication 2, dans lequel la benzo[4,5]thiéno[3,2b]pyran-2-one est présente en mélange avec au moins un autre ingrédient parfumant, solvant ou adjuvant dont l'utilisation convient dans un arôme et/ou une fragrance.

4. Procédé pour améliorer, renforcer ou modifier l'arôme d'une boisson, d'un aliment, d'une composition pharmaceutique, d'un matériau de matrice administrable par voie orale ou d'un autre produit administré par voie orale, comprenant l'addition à celui-ci d'une quantité efficace de benzo[4,5]thiéno[3,2b]pyran-2-one.

5. Composition ou article comprenant de la benzo[4,5]thiéno[3,2b]pyran-2-one et au moins un autre ingrédient parfumant, solvant ou adjuvant dont l'utilisation convient en, parfumerie.

6. Composition selon la revendication 5, dans laquelle la benzo[4,5]thiéno[3,2b]pyran-2-one est présente en une quantité qui est au moins 30 pour cent en poids de la composition.

7. Composition selon la revendication 6, dans laquelle la benzo[4,5]thiéno[3,2b]pyran-2-one est présente en une quantité qui est au moins 60 pour cent en poids de la composition.

8. Parfum ou eau de Cologne, savon, gel pour bain ou douche, shampoing ou autre produit de soin capillaire, réparation cosmétique, déodorant ou antitranspirant corporel, agent de rafraichissement de l'air, détergent ou adoucissant du linge ou agent de nettoyage domestique tout usage comprenant de la benzo[4,5]thiéno[3,2b]pyran-2-one.

9. Déodorant ou antitranspirant corporel, comprenant de la benzo[4,5]thiéno[3,2b]pyran-2-one en tant qu'ingrédient parfumant.

10. Déodorant ou antitranspirant corporel selon la revendication 9, dans lequel la benzo[4,5]thiéno[3,2b]-pyran-2-one est présente en mélange avec au moins un autre ingrédient parfumant, solvant ou adjuvant dont l'utilisation convient en parfumerie.

11. Détergent comprenant de la benzo[4,5]thiéno-[3,2b]pyran-2-one.

12. Détergent selon la revendication 11, dans lequel la benzo[4,5]thiéno[3,2b]pyran-2-one est présente en mélange avec au moins un autre ingrédient parfumant, solvant ou adjuvant d'utilisation courante dans la technique.

13. Composition de blanchiment comprenant de la benzo[4,5]thiéno[3,2b]pyran-2-one.

14. Boisson comprenant de la benzo[4,5]thiéno-[3,2b]pyran-2-one.

15. Boisson selon la revendication 14, choisie dans le groupe constitué par la bière, la liqueur de malt, la limonade et le cola.

16. Aliment, composition pharmaceutique, matériau de matrice administrable par voie orale ou autre produit administré par voie orale comprenant de la benzo[4,5]thiéno[3,2b]pyran-2-one.
